(19) Europäisches Patentamt
European Patent Office
Office européen des brevets

(11) Veröffentlichungsnummer: **0 044 994**
**A1**

# (12) EUROPÄISCHE PATENTANMELDUNG

(21) Anmeldenummer: **81105443.6**

(22) Anmeldetag: **13.07.81**

(51) Int. Cl.³: **C 07 D 405/12**, A 01 N 47/22

(30) Priorität: **25.07.80 DE 3028331**

(43) Veröffentlichungstag der Anmeldung: **03.02.82**
**Patentblatt 82/5**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Heywang, Gerhard, Dr., Nittumer Weg 4, D-5060 Bergisch-Gladbach (DE)**
Erfinder: **Kühle, Engelbert, Dr., Von-Bodelschwingh-Strasse 42, D-5060 Bergisch-Gladbach 2 (DE)**
Erfinder: **Hammann, Ingeborg, Dr., Belfortstrasse 9, D-5000 Koeln 1 (DE)**

(54) **Neue N-carboxylierte N-Methylcarbamate, Verfahren zu ihrer Herstellung und ihre Verwendung als Schädlingsbekämpfungsmittel.**

(57) Die vorliegende Erfindung betrifft neue N-Carbonyl-N-methyl-carbamidsäurearylester der Formel I

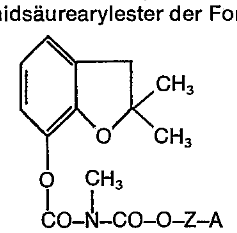

(I)

in welcher

Z für Alkylen,

A für einen gegebenenfalls substituierten, ungesättigten N-haltigen Heterocyclus steht.

Man erhält sie, wenn man 2,3-Dihydro-2,2-dimethyl-7-benzofuranyl-N-chlorcarbonyl-N-methyl-carbamat der Formel II

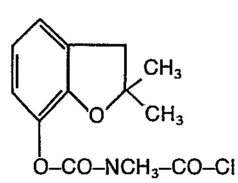

(II)

mit substituierten Alkoholen der Formel III

HO–Z–A (III)

umsetzt.

Die erfindungsgemäßen Verbindungen zeichnen sich durch gute Wirksamkeit als Insektizide, Akarizide und Nematozide aus.

EP 0 044 994 A1

0044994

BAYER AKTIENGESELLSCHAFT 5090 Leverkusen-Bayerwerk
Zentralbereich
Patente, Marken und Lizenzen Rt/ABc
Ia

# Neue N-carboxylierte N-Methylcarbamate, Verfahren zu ihrer Herstellung und ihre Verwendung als Schädlingsbekämpfungsmittel

Die vorliegende Erfindung betrifft neue N-carboxylierte N-Methyl-carbamate, Verfahren zu ihrer Herstellung und ihre Verwendung als Schädlingsbekämpfungsmittel.

Es ist bereits bekannt geworden, N-carboxylierte N-Methyl-carbamidsäurearylester (Vergl. DE-OS 2 132 936) und N-Chlorcarbonyl-N-methylcarbamidsäurearylester (Vergl. DE-OS 2 142 496) insektizide Eigenschaften haben. Ihre Wirkung ist jedoch, vor allem bei niedrigen Aufwandmengen nicht immer voll befriedigend.

Es wurden nun neue N-Carbonyl-N-methylcarbamidsäurearylester der Formel I gefunden,

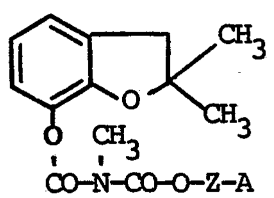

(I)

in welcher

Z für Alkylen

A für einen gegebenenfalls substituierten, ungesättigten N-haltigen Heterocyclus steht.

Die neuen N-Carbonyl-N-methyl-carbamate der Formel I erhält man, wenn man 2,3-Dihydro-2,2-dimethyl-7-benzofuranyl-N-chlorcarbonyl-N-methyl-carbamat der Formel II

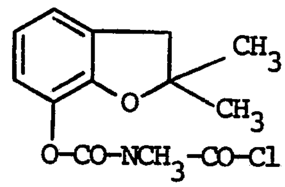

(II)

mit substituierten Alkoholen der Formel III

HO-Z-A (III)

in welcher
Z und A die oben angegebene Bedeutung haben,

gegebenenfalls in Gegenwart eines Verdünnungsmittels und/oder gegebenenfalls in Gegenwart einer Hilfsbase umsetzt.

Die erfindungsgemäßen Verbindungen zeichnen sich durch gute Wirksamkeit als Insektizide, Akarizide und Nematozide aus.

Es ist ausgesprochen überraschend, daß sie bei günstiger Warmblütertoxizität eine höhere Wirkung zeigen als die vom Stand der Technik her bekannten N-carbonylierten N-Methylcarbamate.

Verwendet man 2,3-Dihydro-2,2-dimethyl-7-benzofuranyl-N-chlorcarbonyl-N-methylcarbamat (Formel II) und N-Hydroxymethyl-1,2,4-triazol (Formel IV) als Ausgangsstoffe, so läßt sich der Reaktionsverlauf durch das folgende Formelschema wiedergeben:

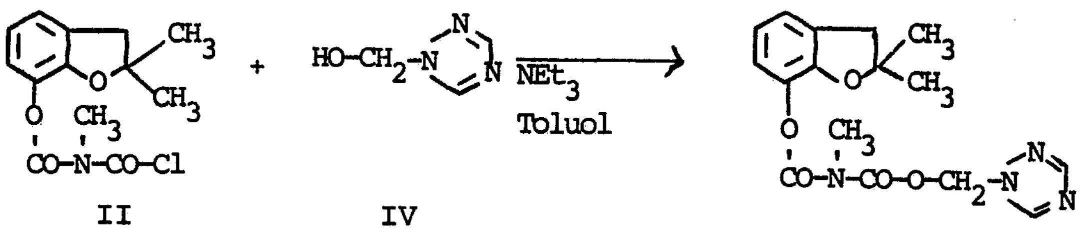


II IV

N-1,2,3-Dihydro-2,2-dimethyl-7-benzofuranyl-N-chlorcarbonyl-N-methylcarbamat (Formel II) und die N-Hydroxyalkylheterocyclen sind bekannt beziehungsweise nach den gängigen Verfahren der chemischen Literatur zugänglich.

Vorzugsweise verwendet werden Hydroxyalkylheterocyclen wie

HO-Z-A (III)

in denen

Z für $C_{1-8}$-Alkylen

und A für $N_{1-3}$-haltiges Azol, das über ein Stickstoffatom an Z gebunden ist, steht.

Besonders bevorzugt werden:

N-(Hydroxymethyl)-pyrazol
N-(2-Hydroxyethyl)-pyrazol
N-(Hydroxymethyl)-1,2,4-triazol
N-(2-Hydroxyethyl)1,2,4-triazol.

Als Verdünnungsmittel eignen sich inerte organische Lösungsmittel. Hierzu gehören Ether wie Diethylether, Dioxan, Tetrahydrofuran, Kohlenwasserstoffe wie Benzol oder Toluol, chlorierte Kohlenwasserstoffe wie Methylenchlorid, Chloroform oder Chlorbenzole, weiterhin Nitrile, Ketone, Ester, sowie Gemische aus diesen Lösungsmitteln.

Als säurebindendes Mittel setzt man dem Reaktionsgemisch Natriumcarbonat, Natriumhydrogencarbonat oder eine tertiäre organische Base, wie z.B. Triethylamin oder Benzyldimethylamin zu.

Die Reaktionstemperatur kann in einem größeren Bereich variiert werden. Im allgemeinen arbeitet man zwischen 0 und 100°C.

Gewöhnlich werden die Reaktionspartner in äquimolaren Mengen eingesetzt, doch auch die Verwendung einer Komponente im Überschuß ist möglich, bringt jedoch keine wesentlichen Vorteile mit sich.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus,

Nephotettix cincticeps, Lecanium corni, Saissetia oleae, Laodelphax striatellus, Nilaparvata lugens, Aonidiella aurantii, Aspidiotus hederae, Pseudococcus spp. Psylla spp. Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma frugiperda, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp.,

Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

0044994

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele:

1. 2,3-Dihydro-2,2-dimethyl-7-benzofuranyl-N-methyl-N-/2-(1,2,4-triazolyl-1)ethoxycarbonyl-7carbamat

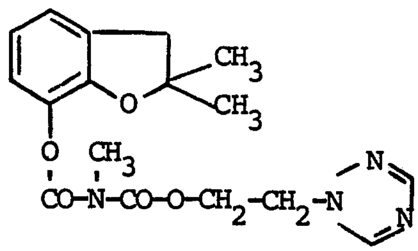

Zu 14,1 g (0,05 mol) 2,3-Dihydro-2,2-dimethyl-7-benzofuranyl-N-chlorcarbonyl-carbamat in 100 ml Toluol werden 5,65 g (0,05 mol) 2-(1,2,4-Triazolyl-1)ethanol und anschließend 4,2 g Natriumhydrogencarbonat eingetragen. Nach dreitägigem Rühren wird das Gemisch mit Wasser (100 ml) versetzt, die organische Phase abgetrennt, getrocknet und im Wasserstrahlpumpenvakuum eingeengt. 15 g farbloses Öl (80 % d. Theorie) $n_D^{20}$ = 1.5372.

Analog werden hergestellt:

2. Öl 46 % $n_D^{20}$=1.5353

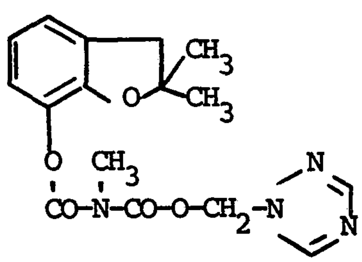

3. 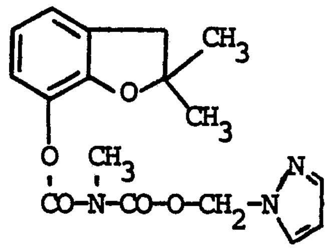 Öl 98 % $n_D^{20}$= 1.5329

Beispiel

Laphygma-Test (Wirkungsdauer nach Spritzen)

| | | |
|---|---|---|
| Lösungsmittel: | 3 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Baumwollpflanzen (Gossypium hirsutum) von ca. 10-15 cm Höhe werden mit der gewünschten Wirkstoffzubereitung tropfnaß gespritzt.

Nach der gewünschten Zeit werden die Pflanzen mit dem Heerwurm (Laphygma frugiperda) besetzt. Nach jeweils 3 Tagen wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1,2.

Patentansprüche

1. N-Carbonyl-N-methylcarbamidsäurearylester der Formel I

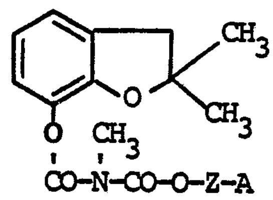

(I)

in welcher

Z für Alkylen

A für einen gegebenenfalls substituierten, ungesättigten N-haltigen Heterocyclus steht.

2. Verfahren zur Herstellung der N-Carbonyl-N-methyl-carbamate der Formel I

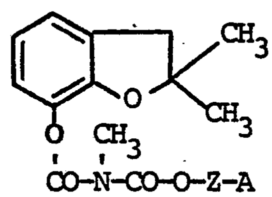

(I)

in welcher

Z für Alkylen

A für einen gegebenenfalls substituierten, ungesättigten N-haltigen Heterocyclus steht,

dadurch gekennzeichnet, daß man 2,3-Dihydro-2,2-dimethyl-7-benzofuranyl-N-chlorcarbonyl-N-methyl-carbamat der Formel II

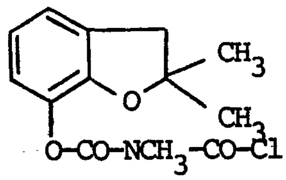

(II)

mit substituierten Alkoholen der Formel III

HO-Z-A (III)

in welcher

Z und A die oben angegebene Bedeutung haben,

gegebenenfalls in Gegenwart eines Verdünnungsmittels und/oder gegebenenfalls in Gegenwart einer Hilfsbase umsetzt.

3. Verbindungen der Formel I, dadurch gekennzeichnet, daß Z für $C_{1-8}$-Alkylen steht.

4. Verbindungen der Formel I, dadurch gekennzeichnet, daß Z und A für N-Methylenpyrazol, N-Methylen-triazol, N-ethylentriazol stehen.

5. Schädlingsbekämpfungsmittel, gekennzeichnet durch einen Gehalt an mindestens einem N-Carbonyl-N-methylcarbamidsäurearylester der Formel I.

6. Verwendung von N-Carbonyl-N-methylcarbamidsäurearylester der Formel I zur Bekämpfung von Schädlingen.

7. Verfahren zur Bekämpfung von Schädlingen, dadurch gekennzeichnet, daß man N-Carbonyl-N-methylcarbamidsäurearylester der Formel I auf Schädlinge und/oder ihren Lebensraum einwirken läßt.

8. Verfahren zur Herstellung von Schädlingsbekämpfungsmitteln, dadurch gekennzeichnet, daß man N-Carbonyl-N-methylcarbamidsäurearylester der Formel I mit Streckmitteln und/oder oberflächenaktiven Mitteln vermischt.

Europäisches Patentamt

**EUROPÄISCHER RECHERCHENBERICHT**

0044994
Nummer der Anmeldung
EP 81 10 5443

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | FR - A - 2 144 695 (BAYER A.G.)<br>* Seite 24; Ansprüche *<br>-- | 1,5 |
| PA | US - A - 4 237 142 (K.H. BUCHEL et al.)<br>* Spalten 1,2 *<br>---- | 1,5 |

KLASSIFIKATION DER ANMELDUNG (Int. Cl.3)

C 07 D 405/12
A 01 N 47/22

RECHERCHIERTE SACHGEBIETE (Int. Cl.3)

C 07 D 405/12
A 01 N 47/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 02-11-1981 | BRIGHENTI |